# FASCICULE DE BREVET EUROPEEN

(11) **EP 3 495 340 B1**
(45) Date de publication et mention de la délivrance du brevet: **29.07.2026**
(21) Numéro de dépôt: 19153219.1
(22) Date de dépôt: 22.06.2015
(51) Int. Cl.: C07C 21/18

(54) **COMPOSITION COMPRENANT DU TETRAFLUOROPROPENE**
ZUSAMMENSETZUNG MIT TETRAFLUORPROPEN
COMPOSITION COMPRISING TETRAFLUOROPROPENE

(30) Priorité: 02.07.2014 FR 1456303
(43) Date de publication de la demande: 12.06.2019
(62) Demande divisionnaire de: 15738402.5
(73) Titulaire: Arkema France, 92800 Puteaux (FR)
(72) Inventeur: WENDLINGER, Laurent, 69493 PIERRE-BENITE CEDEX (FR); PIGAMO, Anne, 69493 PIERRE-BENITE CEDEX (FR); DEUR-BERT, Dominique, 69493 PIERRE-BENITE CEDEX (FR)
(74) Mandataire: Arkema Patent

(56) Documents cités:
- EP-A1- 2 837 613
- WO-A1-2012/098421
- WO-A1-2012/098422
- WO-A1-2013/154059

## Description

### DOMAINE DE L'INVENTION

La présente invention concerne une composition comprenant du tétrafluoropropène caractérisée en ce qu'elle comprend, en proportions molaires, de 1 à 50 ppm de HCC-40, de 1 à 50 ppm de HFC-152a, de 1 à 50 ppm de HFC-41 et de 1 à 50 ppm de HFC-32.

### ARRIERE-PLAN TECHNIQUE

Les gaz à effet de serre sont des composants gazeux qui absorbent le rayonnement infrarouge émis par la surface terrestre, contribuant ainsi à l'effet de serre. L'augmentation de leur concentration dans l'atmosphère est l'un des facteurs à l'origine du réchauffement climatique.

La production des chlorofluorocarbones (CFC) et des hydrochlorofluorocarbures (HCFC) utilisés dans les systèmes de réfrigération et de climatisation a ainsi été successivement réglementée par les protocoles de Montréal puis de Kyoto. Il existe un besoin de développer de nouvelles molécules tout aussi efficaces et présentant en particulier un potentiel de réchauffement global le plus faible possible. C'est le cas des hydrofluorooléfines, et notamment du HFO-1234yf, qui est un composé particulièrement utile.

Il est connu de produire des hydrofluorooléfines ou des hydrofluorocarbures par fluoration d'hydrochlorooléfines ou d'hydrochlorocarbures notamment. Cette fluoration est généralement une fluoration catalytique utilisant l'acide fluorhydrique comme agent fluorant.

La réaction de fluoration doit généralement être effectuée à une température élevée (plus de 300°C), en phase gazeuse, en présence d'un catalyseur solide supporté ou massique.

Il est connu de prévoir une co-alimentation avec un agent oxydant, notamment de l'air, ou éventuellement du chlore, pour préserver la durée de vie du catalyseur et limiter le dépôt de coke à sa surface pendant l'étape réactionnelle.

Le document US 8,614,361 décrit un procédé de fabrication du HFO-1234yf en faisant réagir le HCFO-1233xf avec de l'HF en présence d'une teneur élevée en oxygène.

Le document US 8,618,338 décrit un procédé de fabrication de fluorooléfine en deux étapes, en particulier une première étape de réaction en phase liquide à partir du 1,1,2,3-tétrachloropropène (HCO-1230xa) pour l'obtention de l'intermédiaire HCFO-1233xf et une deuxième étape de réaction en phase gazeuse à partir de HCFO-1233xf pour obtenir du HFO-1234yf.

Le document WO 2013/088195 enseigne un procédé de fabrication de HFO-1234yf en deux étapes, une première étape de fluoration en phase gazeuse de 1,1,1,2,3-pentachloropropane (HCC-240db) et/ou de 1,1,2,2,3-pentachloropropane (HCC-240aa) pour l'obtention de l'intermédiaire HCFO-1233xf, puis une deuxième étape de réaction en phase gazeuse à partir du HCFO-1233xf pour obtenir le HFO-1234yf.

Les documents WO 2012/098421 et WO 2012/098422 enseignent l'activation et la régénération de catalyseurs de fluoration.

Le document WO 2013/182816 décrit un procédé de réaction chimique pour la mise en œuvre alternée d'une phase de réaction catalytique et d'une phase de régénération de catalyseur dans un réacteur.

Il existe encore un besoin d'améliorer les procédés de fabrication des HFO-1234 tels que le HFO-1234yf, et notamment de produire ces composés avec un rendement élevé et dans un degré de pureté élevé.

### RESUME DE L'INVENTION

La présente divulgation concerne en premier lieu un procédé de fabrication de tétrafluoropropène, comprenant, de manière alternée :
- au moins une étape de réaction d'un composé chloré avec de l'acide fluorhydrique en phase gazeuse, en présence d'un catalyseur de fluoration, la proportion d'oxygène éventuellement présent étant inférieure à 0,05 mol.% par rapport au composé chloré ;
- une étape de régénération du catalyseur de fluoration par mise en contact du catalyseur de fluoration avec un flux de régénération comprenant un agent oxydant.

Selon un mode de réalisation, l'étape de réaction du composé chloré avec l'acide fluorhydrique est effectuée essentiellement en l'absence d'oxygène, et de préférence essentiellement en l'absence de tout agent oxydant.

Selon un mode de réalisation, le flux de régénération contient au moins 1 mol.% d'oxygène par rapport au flux de régénération total.

Selon un mode de réalisation, l'étape de réaction du composé chloré avec l'acide fluorhydrique est mise en œuvre dans un réacteur unique, de manière séparée dans le temps par rapport à l'étape de régénération du catalyseur de fluoration.

Selon un mode de réalisation, l'étape de réaction du composé chloré avec l'acide fluorhydrique est mise en œuvre dans au moins un premier réacteur, simultanément à la mise en œuvre de l'étape de régénération du catalyseur de fluoration dans au moins un deuxième réacteur.

Selon un mode de réalisation, le tétrafluoropropène est le 2,3,3,3-tétrafluoropropène.

Selon un mode de réalisation, le tétrafluoropropène est le 1,3,3,3-tétrafluoropropène.

Selon un mode de réalisation, le composé chloré est choisi parmi les tétrachloropropènes, les chlorotrifluoropropènes, les pentachloropropanes et les mélanges de ceux-ci.

Selon un mode de réalisation, le composé chloré est le 2-chloro-3,3,3-trifluoropropène, et le tétrafluoropropène est le 2,3,3,3-tétrafluoropropène.

Selon un mode de réalisation, le composé chloré est le 1,1,1,2,3-pentachloropropane et/ou le 1,1,2,2,3-pentachloropropane, et le tétrafluoropropène est le 2,3,3,3-tétrafluoropropène.

Selon un mode de réalisation, le composé chloré est le 1-chloro-3,3,3-trifluoropropène, et le tétrafluoropropène est le 1,3,3,3-tétrafluoropropène.

Selon un mode de réalisation, le procédé comprend :
- une étape préliminaire de fabrication du composé chloré, qui est de préférence une étape préliminaire de réaction d'un composé préliminaire avec de l'acide fluorhydrique en phase gazeuse, en présence d'un catalyseur de fluoration préliminaire, la proportion d'oxygène éventuellement présent étant inférieure à 0,05 mol.% par rapport au composé préliminaire.

Selon un mode de réalisation, l'étape préliminaire de réaction est effectuée en alternance avec :
- une étape de régénération du catalyseur de fluoration préliminaire par mise en contact du catalyseur de fluoration préliminaire avec un flux de régénération comprenant un agent oxydant.

Selon un mode de réalisation, le composé préliminaire est du 1,1,1,2,3-pentachloropropane et/ou du 1,1,2,2,3-pentachloropropane, le composé chloré est du 1-chloro-3,3,3-trifluoropropène et le tétrafluoropropène est du 2,3,3,3-tétrafluoropropène.

Selon un mode de réalisation, le procédé comprend :
- la collecte d'un flux de produits à l'issue de l'étape préliminaire de réaction ;
- la séparation du flux de produits en un premier flux comprenant de l'acide chlorhydrique et du tétrafluoropropène et un deuxième flux comprenant de l'acide fluorhydrique et le composé chloré ;
- l'utilisation dudit deuxième flux pour effectuer l'étape de réaction du composé chloré avec de l'acide fluorhydrique ; et
- optionnellement, la collecte d'un flux de produits à l'issue de l'étape de réaction du composé chloré avec l'acide fluorhydrique, et le recyclage de celui-ci à l'étape préliminaire de réaction.

La présente divulgation concerne également une installation de fabrication de tétrafluoropropène, comprenant au moins un réacteur de fluoration en phase gazeuse comprenant un lit de catalyseur de fluoration, ledit réacteur de fluoration en phase gazeuse étant configuré pour être alimenté alternativement par :
- un système d'alimentation en flux réactionnel comprenant un composé chloré et de l'acide fluorhydrique, la proportion d'oxygène éventuellement présent dans ce flux réactionnel étant inférieure à 0,05 mol.% par rapport au composé chloré ; et
- un système d'alimentation en flux de régénération comprenant un agent oxydant.

Selon un mode de réalisation, le flux réactionnel est essentiellement dépourvu d'oxygène, et de préférence de tout agent oxydant.

Selon un mode de réalisation, le flux de régénération contient au moins 1 mol.% d'oxygène par rapport au flux de régénération total.

Selon un mode de réalisation, l'installation comprend un unique réacteur configuré pour être alimenté alternativement par le système d'alimentation en flux réactionnel et le système d'alimentation en flux de régénération.

Selon un mode de réalisation, l'installation comprend une pluralité de réacteurs, chacun étant configuré pour être alimenté alternativement par un système d'alimentation en flux réactionnel et un système d'alimentation en flux de régénération.

Selon un mode de réalisation, l'installation est configurée de telle sorte que lorsqu'un réacteur est alimenté par le système d'alimentation en flux réactionnel, un autre réacteur est alimenté par le système d'alimentation en flux de régénération.

Selon un mode de réalisation, l'installation est configurée de telle sorte que :
- le système d'alimentation en flux réactionnel alimente le réacteur en pied et le système d'alimentation en flux de régénération alimente le réacteur en pied ; ou
- le système d'alimentation en flux réactionnel alimente le réacteur en pied et le système d'alimentation en flux de régénération alimente le réacteur en tête ; ou
- le système d'alimentation en flux réactionnel alimente le réacteur en tête et le système d'alimentation en flux de régénération alimente le réacteur en pied ; ou
- le système d'alimentation en flux réactionnel alimente le réacteur en tête et le système d'alimentation en flux de régénération alimente le réacteur en tête.

Selon un mode de réalisation :
- le tétrafluoropropène est le 2,3,3,3-tétrafluoropropène ; ou
- le tétrafluoropropène est le 1,3,3,3-tétrafluoropropène.

Selon un mode de réalisation, le composé chloré est choisi parmi les tétrachloropropènes, les chlorotrifluoropropènes, les pentachloropropanes et les mélanges de ceux-ci ; et de préférence :
- le composé chloré est le 2-chloro-3,3,3-trifluoropropène et le tétrafluoropropène est le 2,3,3,3-tétrafluoropropène ; ou
- le composé chloré est le 1,1,1,2,3-pentachloropropane et/ou le 1,1,2,2,3-pentachloropropane, et le tétrafluoropropène est le 2,3,3,3-tétrafluoropropène ; ou
- le composé chloré est le 1-chloro-3,3,3-trifluoropropène, et le tétrafluoropropène est le 1,3,3,3-tétrafluoropropène.

Selon un mode de réalisation, l'installation comprend :
- au moins une unité de fabrication de composé chloré, qui de préférence est au moins un réacteur préliminaire de fluoration ; configurée pour être alimentée par :
- un système d'alimentation en milieu réactionnel comprenant un composé préliminaire et de l'acide fluorhydrique, la proportion d'oxygène éventuellement présent dans ce flux réactionnel étant inférieure à 0,05 mol.% par rapport au composé préliminaire.

Selon un mode de réalisation, le réacteur préliminaire de fluoration est également configuré pour être alimenté par un système d'alimentation en flux de régénération comprenant un agent oxydant.

Selon un mode de réalisation, le composé préliminaire est le 1,1,1,2,3-pentachloropropane et/ou le 1,1,2,2,3-pentachloropropane, le composé chloré est le 1-chloro-3,3,3-trifluoropropène et le tétrafluoropropène est le 2,3,3,3-tétrafluoropropène.

Selon un mode de réalisation, l'installation comprend :
- au moins un premier réacteur de fluoration catalytique ;
- au moins un deuxième réacteur de fluoration catalytique ;
- un système de collecte de flux de produits connecté en sortie du premier réacteur de fluoration catalytique ;
- une unité de séparation alimentée par le système de collecte de flux de produits ;
- une première conduite de collecte et une deuxième conduite de collecte connectées en sortie de l'unité de séparation, la première conduite de collecte étant configurée pour transporter un flux comprenant de l'acide chlorhydrique et du tétrafluoropropène et la deuxième conduite de collecte étant configurée pour transporter un flux comprenant de l'acide fluorhydrique et du composé chloré ;
- un système de collecte intermédiaire connecté en sortie du deuxième réacteur ;
- un premier système d'alimentation en milieu réactionnel configuré pour alimenter le premier réacteur, celui-ci étant lui-même alimenté par le système de collecte intermédiaire ;
- un deuxième système d'alimentation en milieu réactionnel configuré pour alimenter le deuxième réacteur, celui-ci étant lui-même alimenté par la deuxième conduite de collecte ;
- un système d'alimentation en flux de régénération configuré pour alimenter le premier réacteur et/ou le deuxième réacteur ; et
- un système de collecte de flux de gaz issu de la régénération.

Selon un mode de réalisation, l'installation comporte au moins deux deuxièmes réacteurs configurés de telle sorte que lorsque l'un de ces réacteurs est alimenté par le deuxième système d'alimentation en flux réactionnel, l'autre réacteur est alimenté par le système d'alimentation en flux de régénération.

Selon un mode de réalisation, l'installation comporte au moins deux premiers réacteurs et deux deuxièmes réacteurs configurés de telle sorte que lorsque l'un des premiers réacteurs et l'un des deuxièmes réacteurs sont respectivement alimentés par le premier système d'alimentation en flux réactionnel et le deuxième système d'alimentation en flux réactionnel, l'autre premier réacteur et l'autre deuxième réacteur sont alimentés par le système d'alimentation en flux de régénération ; et qui, de préférence, est configurée de telle sorte qu'un même flux de régénération issu du système d'alimentation en flux de régénération passe successivement dans le premier réacteur puis le deuxième réacteur, ou passe successivement dans le deuxième réacteur puis le premier réacteur.

Selon un mode de réalisation, l'installation comporte un unique deuxième réacteur, configuré pour être alimenté de manière séquentielle soit par le deuxième système d'alimentation en flux réactionnel, soit par le système d'alimentation en flux de régénération.

Selon un mode de réalisation, l'installation comporte un unique premier réacteur et un unique deuxième réacteur, configuré pour être alimenté de manière séquentielle soit par le deuxième système d'alimentation en flux réactionnel, soit par le système d'alimentation en flux de régénération ; et qui, de préférence, est configurée de telle sorte qu'un même flux de régénération issu du système d'alimentation en flux de régénération passe successivement dans le premier réacteur puis le deuxième réacteur, ou passe successivement dans le deuxième réacteur puis le premier réacteur.

L'invention concerne également une composition comprenant du tétrafluoropropène caractérisée en ce qu'elle comprend, en proportions molaires, de 1 à 50 ppm de HCC-40, de 1 à 50 ppm de HFC-152a, de 1 à 50 ppm de HFC-41 et de 1 à 50 ppm de HFC-32.

La présente invention permet de surmonter les inconvénients de l'état de la technique. La présente divulgation fournit plus particulièrement un procédé de fabrication de HFO-1234 (et notamment de HFO-1234yf) ayant un rendement élevé et fournissant le produit souhaité dans un degré de pureté élevé.

Cela est accompli grâce à la découverte par les présents inventeurs que certaines étapes réactionnelles de fluoration peuvent être effectuées essentiellement en l'absence d'agent oxydant tel que l'oxygène, sans que la durée de vie du catalyseur de fluoration ne soit visiblement affectée sur une période déterminée, à condition de prévoir des étapes intermédiaires de régénération.

Il en découle comme avantage l'obtention d'un flux gazeux de HFO-1234 d'une pureté supérieure car obtenu essentiellement en l'absence d'oxygène pendant la réaction. La teneur en oxydes de carbones ainsi qu'en composés contenant un ou deux carbones est nettement réduite par rapport à l'état de la technique. Le traitement aval et la purification finale du produit désiré sont ainsi simplifiés, garantissant l'obtention du produit final de préférence avec une pureté supérieure ou égale à 98 %, avantageusement supérieure ou égale à 99 %, et très avantageusement supérieure ou égale à 99,8 % en poids. L'acide chlorhydrique coproduit est également plus facilement valorisable.

### BREVE DESCRIPTION DES FIGURES

Les **figures 1a** et **1b** représentent de manière schématique un mode de réalisation d'une installation avec un seul réacteur catalytique de fluoration, dans deux configurations de fonctionnement différentes.
Les **figures 2a** et **2b** représentent de manière schématique un mode de réalisation d'une installation avec deux réacteurs catalytiques de fluoration, dans deux configurations de fonctionnement différentes.
La **figure 3** représente de manière schématique un mode de réalisation d'une installation avec trois réacteurs catalytiques de fluoration, dans une configuration de fonctionnement particulière.
Les **figures 4a** et **4b** représentent de manière schématique un mode de réalisation d'une installation avec un seul réacteur catalytique de fluoration, dans deux configurations de fonctionnement différentes.
Les **figures 5a** et **5b** représentent de manière schématique un mode de réalisation d'une installation avec deux réacteurs catalytiques de fluoration, dans deux configurations de fonctionnement différentes.
La **figure 6** représente de manière schématique un mode de réalisation d'une installation avec trois réacteurs catalytiques de fluoration, dans une configuration de fonctionnement particulière.
Les **figures 7** à **11** représentent de manière schématique des modes de réalisation d'installations pour la production de HFO-1234yf en deux étapes.

### DESCRIPTION DE MODES DE REALISATION DE L'INVENTION

L'invention est maintenant décrite plus en détail et de façon non limitative dans la description qui suit.

Sauf mention contraire, les pourcentages et proportions indiqués sont en valeurs massiques.

La présente divulgation prévoit de produire du HFO-1234 par fluoration catalytique en phase gazeuse ; cette fluoration catalytique est, selon l'invention, alternée avec la régénération du catalyseur de fluoration. Dans certains modes de réalisation, l'invention prévoit la production de HFO-1234 en plusieurs étapes de fluoration.

### Réaction de fluoration pour l'obtention de HFO-1234

La présente divulgation prévoit au moins une étape de fluoration, permettant de produire du HFO-1234 à partir d'un composé chloré.

Le HFO-1234 peut être en particulier le HFO-1234yf ou bien le HFO-1234ze (1,3,3,3-tétrafluoropropène), et ce sous forme cis ou trans ou sous forme d'un mélange de formes cis et trans.

Par *« composé chloré »,* on entend un composé organique comprenant un ou plusieurs atomes de chlore. Ce composé comprend de préférence 3 atomes de carbone.

Ce composé chloré est de préférence un propane ou un propène ayant des substituants choisis parmi F, CI, I et Br (de préférence parmi F et CI), et comportant au moins un substituant Cl.

Il est entendu que par « *composé chloré »* on entend également des mélanges de composés.

De préférence, le composé chloré est un tétrachloropropène, un chlorotrifluoropropène, un pentachloropropane ou un mélange de ceux-ci.

Dans un mode de réalisation, le composé chloré est du 2-chloro-3,3,3-trifluoropropène (HCFO-1233xf), pour produire du HFO-1234yf.

Dans un autre mode de réalisation, le composé chloré est 1-chloro-3,3,3-trifluoropropène (HCFO-1233zd), pour produire du HFO-1234ze.

Dans un autre mode de réalisation, le composé chloré est du 1,1,1,2,3-pentachloropropane (HCC-240db), ou du 1,1,2,2,3-pentachloropropane (HCC-240aa), ou encore un mélange des deux, pour produire du HFO-1234yf.

Selon encore un autre mode de réalisation, le composé chloré est du 2,3-dichloro-1,1,1-trifluoropropane (HCFC-243db), pour produire du HFO-1234yf.

Selon encore un autre mode de réalisation, le composé chloré est du 1,1,2,3-tétrachloropropène (HCO-1230xa), ou du 2,3,3,3-tétrachloropropène (HCO-1230xf), ou un mélange de ces deux composés, pour produire du HFO-1234yf.

La conversion du composé chloré en HFO-1234 peut être une conversion directe ou une conversion indirecte (c'est-à-dire faisant intervenir un produit intermédiaire).

La fluoration du composé chloré en HFO-1234 est mise en œuvre dans un ou plusieurs réacteurs de fluoration en phase gazeuse comportant un lit de catalyseur de fluoration.

Le catalyseur utilisé peut être par exemple à base d'un métal comprenant un oxyde de métal de transition ou un dérivé ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, I.1-5 et 28-32), au document EP 939071 (paragraphe [0022]), au document WO 2008/054781 (en p.9 I.22-p.10 I.34), et au document WO 2008/040969 (revendication 1), auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de chrome, et de 0,5 à 20 % en poids de nickel, de préférence de 2 à 10 % de chaque.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, I.30-p.7 I.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF.

Par exemple, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :
- avec un rapport molaire HF / composé chloré de 1:1 à 150:1, de préférence de 3:1 à 100:1 et de manière plus particulièrement préférée de 5:1 à 50:1 ;
- avec un temps de contact de 1 à 100 s, de préférence 1 à 50 s et plus particulièrement 2 à 40 s (volume de catalyseur divisé par le flux entrant total, ajusté à la température et à la pression de fonctionnement) ;
- à une pression absolue allant de 0,1 à 50 bar, de préférence de 0,3 à 15 bar ;
- à une température (température du lit de catalyseur) de 100 à 500°C, de préférence de 200 à 450°C, et plus particulièrement de 250 à 400°C.

Le flux composant le milieu réactionnel peut comporter, outre le HF et le composé chloré, des composés supplémentaires, notamment d'autres halohydrocarbures ou halohydrooléfines.

La durée de l'étape de réaction est typiquement de 10 à 2000 heures, de préférence de 50 à 500 heures et de manière plus particulièrement préférée de 70 à 300 heures.

La proportion d'oxygène éventuellement présent dans le milieu réactionnel est inférieure à 0,05 mol.% par rapport au composé chloré, de préférence encore inférieure à 0,02 mol.% ou inférieure à 0,01 mol.%. Des traces d'oxygène peuvent éventuellement être présentes, mais de préférence l'étape de fluoration est effectuée essentiellement en l'absence d'oxygène, ou en l'absence totale d'oxygène.

De préférence, la proportion de tout agent oxydant (tel que l'oxygène et le chlore) éventuellement présent dans le milieu réactionnel est inférieure à 0,05 mol.% par rapport au composé chloré, de préférence encore inférieure à 0,02 mol.% ou inférieure à 0,01 mol.%. Des traces d'agent oxydant peuvent éventuellement être présentes, mais de préférence l'étape est effectuée essentiellement en l'absence d'agent oxydant, ou en l'absence totale d'agent oxydant.

Le flux de produits issu de l'étape de fluoration du composé chloré en HFO-1234 peut subir des traitements appropriés (distillation, lavage, etc.) afin de récupérer le HFO-1234 sous forme purifiée et le séparer des autres composés présents (HCl, HF non réagi, composé chloré non réagi, autres organiques). Un ou plusieurs flux peuvent faire l'objet d'un recyclage.

Le HCl en particulier peut faire l'objet d'une purification selon le procédé décrit dans la demande n° FR 13/61736, à laquelle il est expressément fait référence.

### Régénération du catalyseur

Dans chaque réacteur utilisé pour la mise en œuvre de la fluoration du composé chloré en HFO-1234, ladite fluoration peut être alternée avec des phases de régénération du catalyseur, en présence d'oxygène.

On peut par exemple passer de la phase de réaction à la phase de régénération lorsque la conversion du composé chloré descend sous un seuil prédéterminé, par exemple de 50 %.

Si besoin, au préalable, une période de transition consistant à décomprimer la phase gaz réactionnelle est assurée. Elle peut être suivie d'une phase de balayage à l'aide d'un gaz inerte ou bien d'une mise sous vide dans le but d'éliminer totalement les réactifs présents.

Le flux de régénération contient de préférence au moins 1 mol.% d'oxygène au total. Il peut s'agir d'air pur mais le flux peut également contenir un gaz inerte utile pour assurer une certaine dilution, par exemple de l'azote, de l'argon, de l'hélium ou bien de l'acide fluorhydrique dans des proportions variant de 0 à 95 %, de préférence de 5 à 85 %, et de manière plus particulièrement préférée de 10 à 80 %. Le débit du flux de régénération est de préférence maintenu suffisamment élevé pour éviter les régimes diffusionnels externes.

La température à l'étape de régénération vaut par exemple de 100 à 500°C, de préférence de 200 à 450°C et de manière plus particulièrement préférée de 250 à 400°C. Il peut être pratique d'effectuer la régénération à la même température que la réaction.

La pression à l'étape de régénération vaut par exemple de la pression atmosphérique à 15 bars absolus. Elle est de préférence approximativement égale à la pression atmosphérique.

La durée de l'étape de régénération est typiquement de 10 à 2000 heures, de préférence de 50 à 500 heures et de manière plus particulièrement préférée de 70 à 300 heures.

La régénération peut s'effectuer en flux co-courant ou contre-courant par rapport au sens du flux utilisé pendant la période de réaction.

Cette étape de régénération permet de recouvrer l'activité initiale du catalyseur. Plusieurs cycles peuvent ainsi être enchaînés sans altérer significativement l'activité du catalyseur, ce qui permet d'augmenter sa durée de vie.

A l'issue de l'étape de régénération, le réacteur peut être mis sous vide de manière à éliminer les gaz inertes et l'oxygène introduits, préalablement à la réintroduction des organiques.

### Installations pour la mise en œuvre de l'étape de fluoration décrite ci-dessus

L'étape de fluoration décrite ci-dessus peut être mise en œuvre avec un réacteur unique. Dans ce cas, celui-ci est opéré de manière alternative en réaction et en régénération. La production est alors discontinue.

Sinon, l'étape de fluoration décrite ci-dessus peut être mise en œuvre avec une pluralité de réacteurs, par exemple deux, trois ou plus de trois réacteurs. Dans ce cas, il est possible d'opérer au moins un réacteur en réaction pendant qu'au moins un autre est opéré en régénération, et ainsi éventuellement d'assurer une production continue.

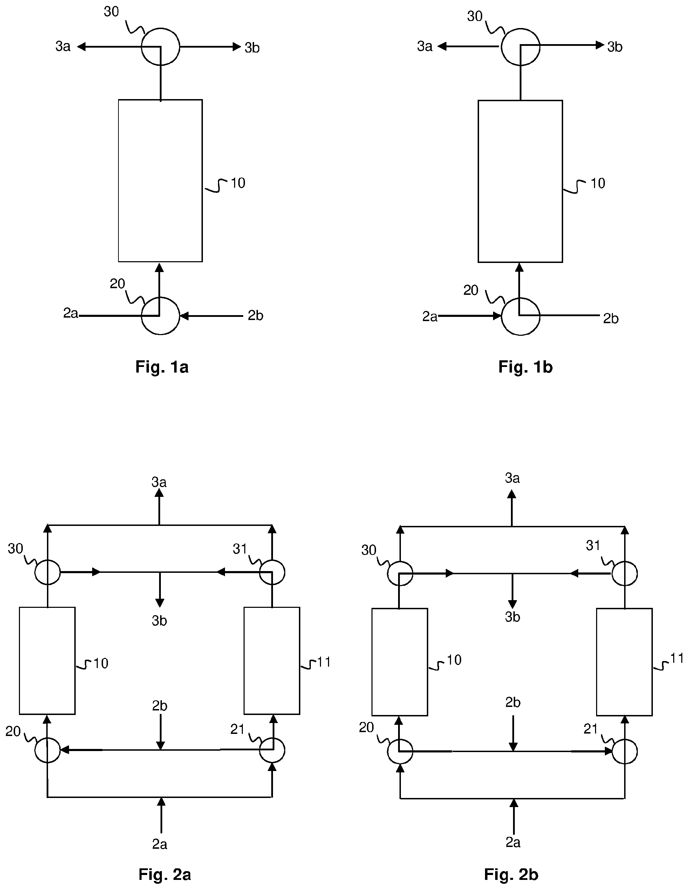

En faisant référence aux **figures 1a** et **1b****,** un mode de réalisation avec un seul réacteur est décrit.

L'installation comporte alors un réacteur 10, susceptible d'être alimenté soit par un système d'alimentation en flux réactionnel 2a, soit par un système d'alimentation en flux de régénération 2b.

En sortie du réacteur 10 sont connectés à la fois un système de collecte de flux de produits 3a et un système de collecte de flux de gaz issu de la régénération 3b.

Par « *système d'alimentation »* et « *système de collecte* », on entend une conduite unique ou un ensemble de plusieurs conduites.

Un système de vannes en entrée 20 et un système de vannes en sortie 30 sont prévus pour permettre de basculer entre les systèmes d'alimentation et de collecte respectifs.

Lors de l'étape de réaction **(****figure 1a****),** le système de vannes en entrée 20 est positionné pour que le réacteur 10 soit alimenté par le système d'alimentation en flux réactionnel 2a ; et le système de vannes en sortie 30 est positionné pour que le réacteur 10 alimente le système de collecte de flux de produits 3a, qui dirige le flux de produits vers des unités de traitement aval des gaz de production.

Lors de l'étape de régénération **(****figure 1b****),** le système de vannes en entrée 20 est positionné pour que le réacteur 10 soit alimenté par le système d'alimentation en flux de régénération 2b ; et le système de vannes en sortie 30 est positionné pour que le réacteur 10 alimente le système de collecte de flux de gaz issu de la régénération 3b, qui dirige le flux de gaz issu de la régénération vers des unités de traitement aval de ces gaz.

Le réacteur 10 enchaîne alternativement des périodes de production et de régénération de manière séquentielle. La production est discontinue.

En faisant référence aux **figures 2a** et **2b****,** un mode de réalisation avec deux réacteurs est maintenant décrit.

Dans une première configuration **(****figure 2a****),** l'étape de réaction est effectuée dans un premier réacteur 10 et l'étape de régénération est effectuée dans un deuxième réacteur 11. Dans une deuxième configuration **(****figure 2b****),** l'étape de réaction est effectuée dans le deuxième réacteur 11 et l'étape de régénération est effectuée dans le premier réacteur 10. De cette façon, la production est continue.

Chaque réacteur 10, 11 est pourvu d'un système de vannes en entrée 20, 21 respectif ainsi que d'un système de vannes en sortie 30, 31 respectif afin de permettre le passage d'une configuration à l'autre. On peut prévoir que le système d'alimentation en flux réactionnel 2a, le système d'alimentation en flux de régénération 2b, le système de collecte de flux de produits 3a et le système de collecte de flux de gaz issu de la régénération 3b soient communs pour les deux réacteurs 10, 11, comme illustré, ou bien prévoir des systèmes distincts dédiés à chaque réacteur 10, 11.

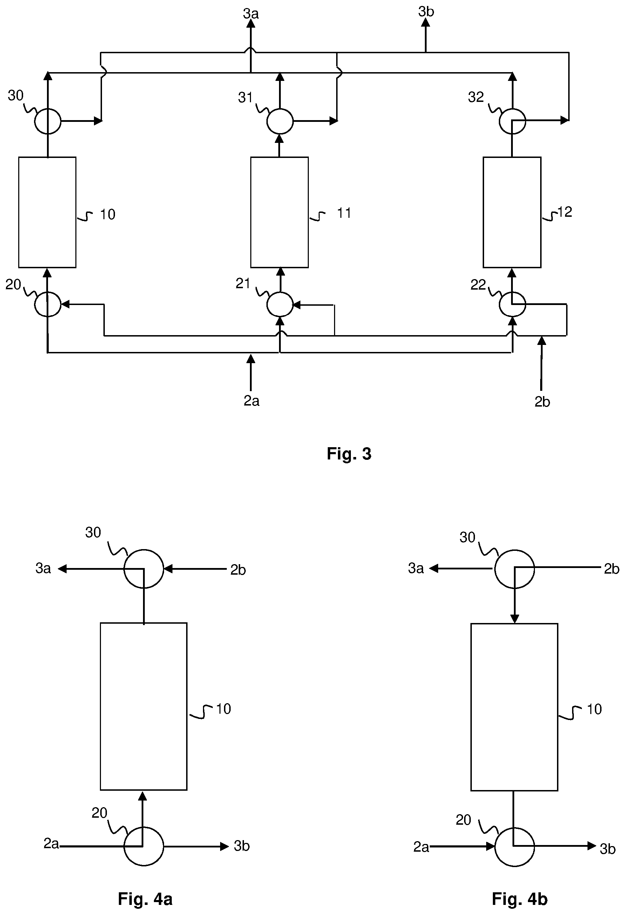

En faisant référence à la **figure 3****,** un mode de réalisation avec trois réacteurs est maintenant décrit.

Dans la configuration illustrée, l'étape de réaction est effectuée dans un premier réacteur 10, un deuxième réacteur 11 est en attente, et l'étape de régénération est effectuée dans un troisième réacteur 12. L'étape d'attente est un état dans lequel le réacteur a été régénéré et est prêt pour repartir en réaction. Dans d'autres configurations non illustrées, on opère une commutation des états des réacteurs 10, 11, 12. De cette façon, on peut assurer une production continue.

Chaque réacteur 10, 11, 12 est pourvu d'un système de vannes en entrée 20, 21, 22 respectif ainsi que d'un système de vannes en sortie 30, 31, 32 respectif afin de permettre le passage d'une configuration à l'autre. On peut prévoir que le système d'alimentation en flux réactionnel 2a, le système d'alimentation en flux de régénération 2b, le système de collecte de flux de produits 3a et le système de collecte de flux de gaz issu de la régénération 3b soient communs pour les trois réacteurs 10, 11, 12 comme illustré, ou bien prévoir des systèmes distincts dédiés à chaque réacteur 10, 11, 12.

Dans les modes de réalisation des **figures 1a, 1b, 2a, 2b** et **3****,** les flux dans les réacteurs sont orientés dans la même direction pour la fluoration et pour la régénération.

Selon des variantes, les flux dans les réacteurs peuvent être orientés en sens inverse entre la fluoration et la régénération.

Ainsi, en **figures 4a** et **4b** est représenté un mode de réalisation avec un seul réacteur 10, qui est analogue au mode de réalisation des **figures 1a** et **1b****,** à la différence que les flux sont inversés entre la fluoration et la régénération. Par exemple, si le système d'alimentation en flux réactionnel 2a alimente le réacteur 10 en pied, alors le système d'alimentation en flux de régénération 2b alimente le réacteur 10 en tête (ou vice versa). De même, si le système de collecte de flux de produits 3a est connecté en tête du réacteur 10, alors le système de collecte de flux de gaz issu de la régénération 3b est connecté en pied du réacteur 10 (ou vice versa).

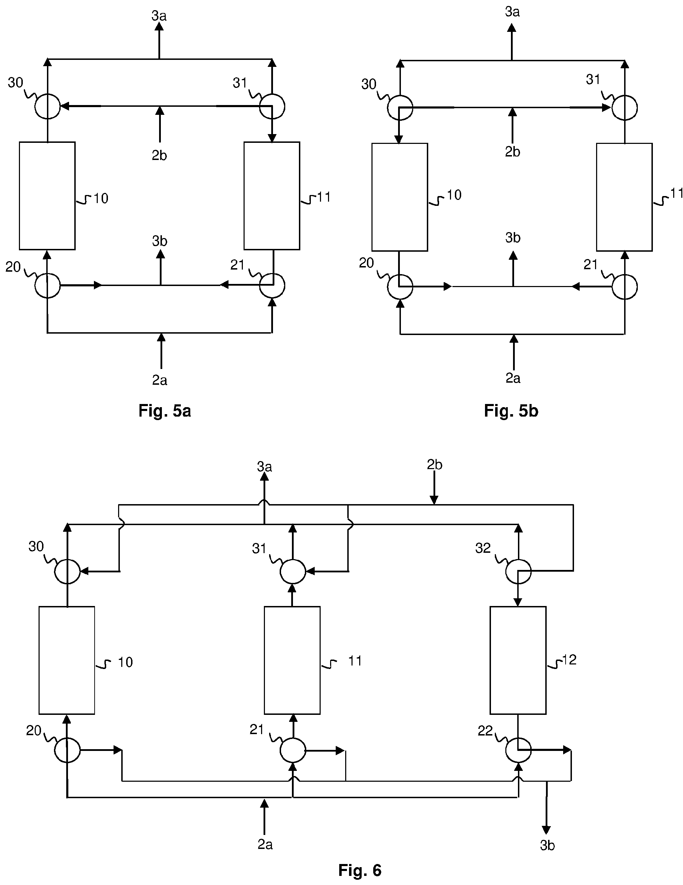

De même, en **figures 5a** et **5b** est représenté un mode de réalisation avec deux réacteurs 10, 11, qui est analogue au mode de réalisation des **figures 2a** et **2b****,** à la différence que les flux sont inversés entre la fluoration et la régénération. Par exemple, si le système d'alimentation en flux réactionnel 2a alimente les réacteurs 10, 11 en pied, alors le système d'alimentation en flux de régénération 2b alimente les réacteurs 10, 11 en tête (ou vice versa). De même, si le système de collecte de flux de produits 3a est connecté en tête des réacteurs 10, 11, alors le système de collecte de flux de gaz issu de la régénération 3b est connecté en pied des réacteurs 10, 11 (ou vice versa).

De même, en **figure 6** est représenté un mode de réalisation avec trois réacteurs 10, 11, 12, qui est analogue au mode de réalisation de la **figure 3****,** à la différence que les flux sont inversés entre la fluoration et la régénération. Par exemple, si le système d'alimentation en flux réactionnel 2a alimente les réacteurs 10, 11, 12 en pied, alors le système d'alimentation en flux de régénération 2b alimente les réacteurs 10, 11, 12 en tête (ou vice versa). De même, si le système de collecte de flux de produits 3a est connecté en tête des réacteurs 10, 11, 12 alors le système de collecte de flux de gaz issu de la régénération 3b est connecté en pied des réacteurs 10, 11, 12 (ou vice versa).

### Procédés en plusieurs étapes

Dans certains modes de réalisation, la présente divulgation prévoit plusieurs étapes réactionnelles successives, et de manière préférée : d'abord une étape préliminaire de fabrication du composé chloré mentionné ci-dessus ; puis ensuite l'étape de fluoration du composé chloré en HFO-1234.

De préférence, l'étape préliminaire est elle-même une étape de fluoration.

Dans ce cas, cette étape convertit un composé préliminaire en le composé chloré mentionné ci-dessus. Dans un tel cas, il faut noter que le composé chloré comporte au moins un atome de fluor (puisqu'il est issu d'une étape de fluoration) ainsi qu'au moins un atome de chlore (puisqu'il est ensuite soumis à l'étape de fluoration décrite ci-dessus pour fournir du HFO-1234).

Le *« composé préliminaire »* est avantageusement un composé organique (de préférence à 3 atomes de carbone) qui comprend au moins deux atomes de chlore (et qui comprend plus d'atomes de chlore que le *« composé chloré »*)*.*

Le composé préliminaire peut être de préférence un propane ou un propène ayant des substituants choisis parmi F, Cl, I et Br (de préférence parmi F et CI), et comportant au moins deux substituants Cl. Un propane est plus particulièrement préféré.

Il est entendu que par *« composé préliminaire »* on entend également des mélanges de composés.

Selon un mode de réalisation préféré, le composé préliminaire est du HCC-240db ou du HCC-240aa, ou encore un mélange des deux, et le composé chloré est du HCFO-1233xf, pour produire du HFO-1234yf.

Selon encore un autre mode de réalisation, le composé préliminaire est du HCFC-243db, et le composé chloré est du HCFO-1233xf, pour produire du HFO-1234yf.

Selon encore un autre mode de réalisation, le composé préliminaire est du HCO-1230xa ou du HCO-1230xf ou un mélange de ces deux composés, et le composé chloré est du HCFO-1233xf, pour produire du HFO-1234yf.

La conversion du composé préliminaire en le composé chloré peut être une conversion directe ou une conversion indirecte (c'est-à-dire faisant intervenir un produit intermédiaire).

Il est possible d'effectuer la fluoration du composé préliminaire en composé chloré en phase liquide. Mais, de préférence, il s'agit d'une fluoration en phase gazeuse, en présence d'un catalyseur de fluoration. Elle peut être mise en œuvre dans un ou plusieurs réacteurs de fluoration en série ou en parallèle.

Le catalyseur de fluoration peut être du même type que décrit ci-dessus pour la fluoration du composé chloré en HFO-1234. La description ci-dessus concernant l'activation du catalyseur s'applique également.

La réaction de fluoration en phase gazeuse du composé préliminaire en composé chloré peut notamment être effectuée :
- avec un rapport molaire HF / organiques de 3:1 à 100:1, de préférence de 5:1 à 50:1 (le terme « *organiques »* désigne l'ensemble des composés du milieu réactionnel comportant un ou plusieurs atomes de carbone) ;
- à une pression absolue allant de 0,1 à 50 bar, de préférence de 0,3 à 15 bar ;
- avec un temps de contact de 1 à 100 s, de préférence de 1 à 50 s et plus particulièrement 2 à 40 s (volume de catalyseur divisé par le flux entrant total, ajusté à la température et à la pression de fonctionnement) ;
- à une température (température du lit de catalyseur) de 100 à 500°C, de préférence de 200 à 450°C, et plus particulièrement de 250 à 400°C.

Le flux composant le milieu réactionnel peut comporter, outre le HF et le composé préliminaire, des composés supplémentaires, notamment d'autres halohydrocarbures ou halohydrooléfines. Le flux peut par exemple déjà comprendre une fraction de HFO-1234.

Selon un mode de réalisation préféré, il n'y a pas ou essentiellement pas d'oxygène (et éventuellement, il n'y a pas ou essentiellement pas d'autre agent oxydant) dans le milieu réactionnel.

Ainsi, on évite également la présence d'oxygène ou d'agent oxydant dans l'étape ultérieure de fluoration, sans avoir à effectuer une séparation intermédiaire d'un flux d'oxygène ou agent oxydant.

La durée de l'étape de réaction du composé préliminaire en composé chloré est typiquement de 10 à 2000 heures, de préférence de 50 à 500 heures et de manière plus particulièrement préférée de 70 à 300 heures.

A l'issue de cette étape de réaction, on collecte un flux de produits qui comprend notamment du composé chloré, du composé préliminaire non réagi, du HF, du HCl, éventuellement du HFO-1234, et éventuellement des produits secondaires comme notamment le 1,1,1,2,2-pentafluoropropane (HFC-245cb).

Ce flux de produits peut ensuite alimenter directement l'étape de fluoration du composé chloré en HFO-1234yf, décrite ci-dessus.

Alternativement, ce flux de produits peut être séparé, par exemple par distillation, pour fournir par exemple un premier flux comprenant du HCl et éventuellement du HFO-1234, et un deuxième flux comprenant du HF et du composé chloré. La distillation peut par exemple être effectuée à une température de -90 à 150°C, de préférence de -85 à 100°C et à une pression de 0,1 à 50 bar abs et de préférence de 0,3 à 5 bar abs.

Le premier flux peut être dirigé vers une unité de production d'acide pour produire du HCl et du HFO-1234. Le HFO-1234 et les produits intermédiaires peuvent être récupérés par des moyens connus tels que des moyens d'extraction, de lavage, de décantation et de préférence de distillation.

Il faut noter que au moins l'une des deux étapes de fluoration décrites dessus est alternée avec une étape de régénération du ou des réacteurs avec un flux d'agent oxydant, comme décrit ci-dessus en lien avec la fluoration du composé chloré en HFO-1234. La description ci-dessus s'applique donc par analogie (y compris celle relative aux différentes installations possibles illustrées dans les **figures 1a** à **6****)** :
- soit à la régénération alternée avec la fluoration du composé préliminaire en composé chloré ;
- soit à la régénération alternée avec la fluoration du composé chloré en HFO-1234 ;
- soit à la fois à la régénération alternée avec la fluoration du composé préliminaire en composé chloré et à la régénération alternée avec la fluoration du composé chloré en HFO-1234.

Selon les conditions de réaction et la nature du catalyseur, la tendance du catalyseur à se désactiver peut être différente, d'où ces divers cas de figures possibles.

### Procédés de fabrication de HFO-1234yf en deux étapes

A présent, on décrit divers modes de réalisation en relation avec la fabrication de HFO-1234yf en deux étapes à partir de HCC-240db (étant entendu qu'on peut également utiliser à la place du HCC-240aa ou un mélange des deux) : une première étape de conversion du HCC-240db en HCFO-1233xf, puis une deuxième étape de conversion du HCFO-1233xf en HFO-1234yf, mises en œuvre dans des réacteurs successifs.

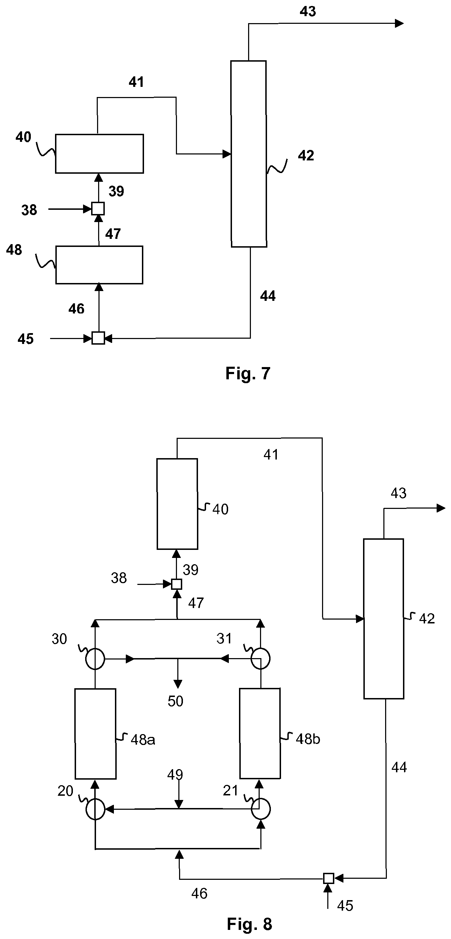

En faisant référence à la **figure 7****,** selon un mode de réalisation, une installation peut ainsi comprendre un premier réacteur de fluoration 40 pour la mise en œuvre de l'étape de préparation du HCFO-1233xf. Il est entendu qu'on peut également utiliser à la place une pluralité de réacteurs, fonctionnant en série et / ou en parallèle.

Ce premier réacteur de fluoration 40 est alimenté par un premier système d'alimentation 39 en milieu réactionnel (comprenant du HF et du HCC-240db).

En sortie du premier réacteur de fluoration 40, on dispose un système de collecte de flux de produits 41, qui alimente une unité de séparation 42. Cette unité de séparation 42 peut être notamment une unité de distillation telle que décrite ci-dessus.

En sortie de l'unité de séparation 42, on prévoit une première conduite de collecte 43 et une deuxième conduite de collecte 44. La première conduite de collecte 43 est configurée pour transporter un flux comprenant notamment du HCl et du HFO-1234yf, et la deuxième conduite de collecte 44 est configurée pour transporter un flux comprenant notamment du HF et du HCFO-1233xf.

La première conduite de collecte 43 alimente des unités de traitement complémentaires non représentées, qui peuvent notamment comprendre une unité de production d'acide, tandis que la deuxième conduite de collecte 44 assure un recyclage vers au moins un deuxième réacteur de fluoration en phase gazeuse 48 qui est utilisé pour la fluoration du HCFO-1233xf en HFO-1234yf. Cette deuxième conduite de collecte 44 peut donc être aussi qualifiée de conduite de recyclage. Ce deuxième réacteur 48 est alimenté par un deuxième système d'alimentation 46 en milieu réactionnel, qui lui-même est alimenté par la deuxième conduite de collecte 44 d'une part et par un système d'alimentation en HF 45 d'autre part.

En sortie du deuxième réacteur 48 est connecté un système de collecte intermédiaire 47. Celui-ci alimente à son tour le premier système d'alimentation 39 en milieu réactionnel du premier réacteur 40. Un apport de HCC-240db est assuré par un système d'alimentation en HCC-240db 38.

De préférence, dans cette installation et dans l'ensemble des étapes de fluoration, la proportion d'oxygène éventuellement présent dans les flux est inférieure à 0,05 mol.% par rapport au composé organique majoritaire, de préférence encore inférieure à 0,02 mol.% ou inférieure à 0,01 mol.%. Des traces d'oxygène peuvent éventuellement être présentes, mais de préférence l'ensemble du procédé de fluoration du HCC-240db en HFO-1234yf est effectué essentiellement en l'absence d'oxygène, ou en l'absence totale d'oxygène.

De préférence, la proportion de tout agent oxydant (tel que l'oxygène et le chlore) éventuellement présent dans le milieu réactionnel est inférieure à 0,05 mol.% par rapport au composé organique majoritaire, de préférence encore inférieure à 0,02 mol.% ou inférieure à 0,01 mol.%. Des traces d'agent oxydant peuvent éventuellement être présentes, mais de préférence l'ensemble du procédé de fluoration du HCC-240db en HFO-1234yf est effectué essentiellement en l'absence d'agent oxydant, ou en l'absence totale d'agent oxydant.

Une régénération du catalyseur est prévue, en alternance avec la fluoration. Celle-ci peut concerner soit le premier réacteur 40, soit le deuxième réacteur 48, soit les deux réacteurs 40, 48. La régénération est effectuée comme décrit ci-dessus, au moyen d'un flux d'agent oxydant. Les moyens nécessaires à la régénération ne sont pas représentés sur la **figure 7** mais sont analogues à ceux décrits ci-dessus.

En **figure 8** est illustrée une variante. Celle-ci est identique au mode de réalisation de la **figure 7** à la différence qu'on prévoit, à la place d'un unique deuxième réacteur 48, deux deuxièmes réacteurs 48a, 48b. Ceux-ci sont configurés pour fonctionner en alternance en mode fluoration et en mode régénération, comme décrit ci-dessus en lien avec les **figures 2a** et **2a****.**

Ainsi, en contrôlant un système de vannes en entrée 20, 21 et un système de vannes en sortie 30, 31, on fait en sorte que :
- dans une phase, l'un des deuxièmes réacteurs 48a fonctionne en mode fluoration, c'est-à-dire est alimenté par le deuxième système d'alimentation 46 en milieu réactionnel et alimente le système de collecte intermédiaire 47 ; tandis que l'autre des deuxièmes réacteurs 48b fonctionne en mode régénération, c'est-à-dire est alimenté par un système d'alimentation en flux de régénération 49 et alimente lui-même un système de collecte de flux de gaz issu de la régénération 50 ;
- dans une autre phase, les configurations des deux réacteurs 48a, 48b sont inversées.

Il faut noter que, sur la **figure 8****,** on a représenté une régénération s'effectuant dans le même sens que la fluoration. Toutefois, les flux peuvent également être inversés, comme décrit en lien avec les **figures 5a** et **5b****.**

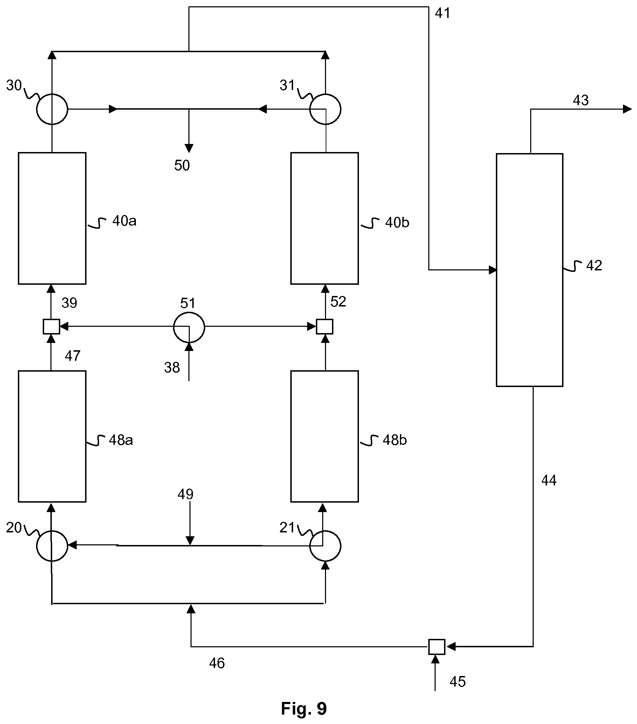

En **figure 9** est illustrée une autre variante. Celle-ci est identique au mode de réalisation de la **figure 8** à la différence qu'on prévoit non seulement deux deuxièmes réacteurs 48a, 48b, mais également, à la place d'un unique premier réacteur, deux premiers réacteurs 40a, 40b. Ceux-ci sont configurés pour fonctionner en alternance en mode fluoration et en mode régénération, comme décrit ci-dessus en lien avec les **figures 2a** et **2b****.**

Dans une première phase, qui est celle illustrée sur la figure, le deuxième système d'alimentation 46 en milieu réactionnel alimente l'un des deux deuxièmes réacteurs 48a. Le système de collecte intermédiaire 47 est connecté en sortie de ce deuxième réacteur 48a, ce qui permet de collecter un flux de produits intermédiaire. Celui-ci alimente le premier système d'alimentation 39 en milieu réactionnel (avec également le système d'alimentation en HCC-240db 38), qui lui-même alimente l'un des deux premiers réacteurs 40a.

Le système de collecte de flux de produits 41 est connecté en sortie de ce premier réacteur 40a.

De préférence simultanément, le système d'alimentation en flux de régénération 49 alimente l'autre deuxième réacteur 48b. Un système de collecte intermédiaire de flux de gaz issu de la régénération 52 est connecté en sortie de ce deuxième réacteur 48b et alimente en entrée l'autre premier réacteur 40b. Le système de collecte intermédiaire de flux de gaz issu de la régénération 50 est connecté en sortie de ce premier réacteur 40b

Alternativement, on peut prévoir une alimentation intermédiaire en flux de régénération supplémentaire entre les deux réacteurs 48b, 40b. Alternativement encore, on peut prévoir une régénération par des flux indépendants de ces deux réacteurs 48b, 40b.

Alternativement encore, on peut prévoir une régénération avec des flux en sens inverse de ceux de la fluoration, selon les principes des **figures 5a** et **5b****.**

Dans une deuxième phase non illustrée, les configurations de fluoration et de régénération sont inversées entre les réacteurs.

Le passage d'une configuration à l'autre est assuré au moyen d'un ensemble des vannes : dans l'exemple illustré, il d'agit de vannes en entrée 20, 21 qui sont situées en amont des deuxièmes réacteurs 48a, 48b, de vannes en sortie qui sont situées en aval des premiers réacteurs 40a, 40b, et enfin d'une vanne de HCC-240db 51 située au niveau du système d'alimentation en HCC-240db 38.

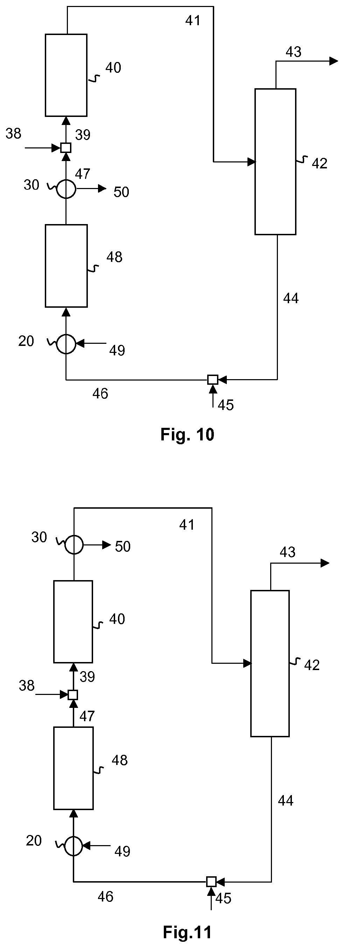

En **figure 10** est illustrée une autre variante. Celle-ci est analogue au mode de réalisation de la **figure 7****.** Dans cette variante, on prévoit une régénération séquentielle par rapport à la fluoration (et non pas simultanée), sur un seul des deux réacteurs, à savoir le deuxième réacteur 48.

A cet effet, on connecte le système d'alimentation en flux de régénération 49 en entrée du deuxième réacteur 48 et le système de collecte de flux de gaz issu de la régénération 50 en sortie du deuxième réacteur 48. Un système de vannes en entrée 20 et un système de vannes en sortie 30 permet d'assurer le basculement du deuxième réacteur 48 soit en fluoration soit en régénération.

Il faut noter que les flux en fluoration et en régénération peuvent être de même sens ou de sens opposé.

Il faut noter encore que l'on peut également prévoir les mêmes moyens pour assurer la régénération au niveau du premier réacteur 40, soit en plus, soit en remplacement, des moyens de régénération du deuxième réacteur 48.

En **figure 11** est illustrée une autre variante. Celle-ci est analogue au mode de réalisation de la **figure 7****.** Dans cette variante, on prévoit une régénération séquentielle par rapport à la fluoration (et non pas simultanée), sur les deux réacteurs à la fois, à savoir le premier réacteur 40 et le deuxième réacteur 48.

A cet effet, on connecte le système d'alimentation en flux de régénération 49 en entrée du deuxième réacteur 48 et le système de collecte de flux de gaz issu de la régénération 50 en sortie du premier réacteur 40. Un système de vannes en entrée 20 et un système de vannes en sortie 30 permet d'assurer le basculement des réacteurs 40, 48 soit en fluoration soit en régénération.

Il faut noter que les flux en fluoration et en régénération peuvent être de même sens ou de sens opposé.

Tout ce qui a été décrit ici en lien avec la préparation du HFO-1234yf en deux étapes peut être lu de manière analogue en remplaçant le HCC-240db par un autre composé préliminaire de départ (et en remplaçant le HCFO-1233xf par un autre composé chloré). De même, ce qui a été décrit ici peut s'appliquer de manière analogue à la préparation d'autres HFO-1234.

Une autre possibilité de mise en œuvre de présente divulgation consiste à : d'une part produire du composé chloré à partir du composé préliminaire (par exemple du HCFO-1233xf à partir de HCC-240db ou analogue) ; et d'autre part produire du HFO-1234 à partir du composé chloré (par exemple du HFO-1234yf à partir de HCFO-1233xf) ; et ce de manière indépendante et séparée, par exemple en isolant, stockant et/ou transportant le composé chloré entre les deux étapes ; et en pratiquant la régénération alternée selon la présente divulgation sur la première étape ou la deuxième étape ou les deux, de manière indépendante.

### Produits obtenus

La conséquence de l'absence ou quasi-absence d'oxygène pendant la phase réactionnelle est la diminution du taux d'impuretés liées aux réactions de combustion ou de dégradation des molécules. Les impuretés sont les oxydes ou dioxydes de carbone ainsi que les molécules contenant moins d'atomes de carbone que le produit chloré de départ.

Ainsi, le procédé de la présente divulgation permet d'obtenir un flux de HFO-1234 (et notamment de HFO-1234yf) contenant moins de chlorométhane (HCC-40) et de 1,1-difluoroéthane (HFC-152a) que dans l'état de la technique. Or ces composés forment un azéotrope avec le HFO-1234yf, ce qui les rend difficiles à purifier.

Le procédé de la présente divulgation permet également d'obtenir un flux de HFO-1234 (et notamment de HFO-1234yf) contenant moins de fluorométhane (HFC-41) et de difluorométhane (HFC-32) que dans l'état de la technique. Or il est connu que ces composés sont extrêmement inflammables.

La proportion molaire de chacun de ces composés dans le flux de HFO-1234 est ainsi de préférence inférieure à 100 ppm, et plus particulièrement inférieure à 50 ppm.

Selon un mode de réalisation du procédé de la présente divulgation ce flux contient du HFO-1234 (de préférence HFO-1234yf) ainsi que de 1 à 50 ppm de HCC-40, de 1 à 50 ppm de HFC-152a, de 1 à 50 ppm de HFC-41 et de 1 à 50 ppm de HFC-32.

Selon un mode de réalisation, ce flux est essentiellement dépourvu, et de préférence est dépourvu, de HCC-40.

Selon un mode de réalisation, ce flux est essentiellement dépourvu, et de préférence est dépourvu, de HFC-152a.

Selon un mode de réalisation, ce flux est essentiellement dépourvu, et de préférence est dépourvu, de HFC-41.

Selon un mode de réalisation, ce flux est essentiellement dépourvu, et de préférence est dépourvu, de HFC-32.

Selon un mode de réalisation, ce flux contient au moins 98 % de HFO-1234, de préférence au moins 99 %, et notamment au moins 99,5 % voire même au moins 99,8 % en poids.

Le flux de HFO-1234 considéré est soit le flux obtenu en sortie du réacteur de fluoration du composé chloré en HFO-1234 (flux prélevé dans le système de collecte de flux de produit 3a dans les figures), soit le flux obtenu en sortie de l'unité de séparation (flux prélevé dans la première conduite de collecte 43 dans les figures), soit le flux obtenu ultérieurement encore après séparation du HFO-1234 et de l'acide chlorhydrique.

Par ailleurs, l'absence ou quasi-absence d'oxygène permet également l'obtention d'un flux d'acide chlorhydrique d'une pureté supérieure permettant une valorisation plus aisée. Ainsi, le flux d'acide chlorhydrique récupéré après séparation d'avec le HFO-1234 est de préférence dépourvu (ou essentiellement dépourvu) d'acide trifluoroacétique, de COF₂ ou de COFCI.

### EXEMPLES

Les exemples suivants illustrent l'invention sans la limiter.

On dispose d'un réacteur de fluoration en phase gazeuse équipé d'une alimentation en HF, d'une alimentation en produits organiques frais, d'une alimentation disponible pour la co-alimentation d'un autre composé gazeux et d'une conduite d'alimentation issue du recyclage des réactifs non convertis.

La sortie du flux gazeux issu de ce réacteur est menée vers une conduite refroidie avec une double enveloppe qui permet de refroidir et de condenser partiellement les produits de réaction avant leur introduction dans la colonne à distiller. Le flux partiellement condensé est ainsi amené vers une colonne à distiller de hauteur 1,5 m remplie d'un garnissage métallique de type Sulzer qui facilite les échanges entre le flux gazeux montant et le reflux liquide descendant. La colonne à distiller est équipée d'un bouilleur en pied de colonne et d'un système de condensation en tête. Cette unité de séparation permet de séparer un flux en tête constitué majoritairement du produit voulu (HFO-1234yf) et du sous-produit HCl. Des quantités plus ou moins importantes de sous-produit HFC-245cb sont également présentes. Le flux en pied de colonne est majoritairement constitué de HF et de réactif non converti (HCFO-1233xf) ainsi que du sous-produit HFC-245cb issu de l'addition du HF sur le HFO-1234yf. Ce flux en pied de colonne est ensuite recyclé vers le réacteur phase gaz. Des traces d'impuretés sont présentes dans chacun des flux.

180 mL de catalyseur massique à base de chrome sont introduits dans le réacteur en inconel. Il est d'abord soumis à une période de séchage sous 50 L/h d'azote à pression atmosphérique à 275°C pendant une nuit. Ensuite, tout en maintenant l'azote et toujours à 275°C, un flux de HF est ajouté progressivement jusqu'à obtenir un débit de 1 mol/h. Ce traitement est maintenu pendant une nuit. L'azote est ensuite coupé et la température du four augmentée à 350°C. Le traitement sous HF pur est ainsi maintenu une nuit également. Finalement, un traitement sous 5 L/h d'air est appliqué pendant au moins 24 h.

A la suite du traitement d'activation du catalyseur, les réactifs HCFO-1233xf et HF sont introduits dans la boucle de recyclage de manière à remplir cette partie de l'installation en conservant un ratio molaire entre l'acide fluorhydrique et l'organique de 25. Le démarrage se fait en alimentant le liquide contenu dans la boucle de recyclage vers le réacteur en phase gaz (un préchauffeur assure la vaporisation préalable des réactifs). Le système s'équilibre ensuite progressivement entre les réactifs non convertis qui sont recyclés, les produits formés qui sont évacués et collectés hors du système et les réactifs frais qui sont alimentés en continu de manière à compenser exactement la quantité de produits évacués. Le niveau de liquide dans la colonne à distiller reste ainsi constant.

La conversion du catalyseur évolue dans le temps et décroit progressivement. Lorsque la conversion chute en-dessous de 50 %, un traitement de régénération à l'air est appliqué au catalyseur. Ce traitement permet de récupérer intégralement l'activité initiale du catalyseur.

La conversion est calculée à partir de la teneur molaire en HCFO-1233xf mesurée à l'entrée du réacteur (somme des flux de recyclage et d'organique frais) et de la teneur en HCFO-1233xf mesurée à la sortie du réacteur.

### Exemple 1 - résultats catalytiques en présence d'air

Un essai est réalisé dans les conditions opératoires suivantes : le catalyseur est fraîchement régénéré, le ratio molaire entre l'HF et les organiques et de 25, le temps de contact en phase gaz est de 15 secondes, la température est de 350°C et on ajoute 10 mol.% d'oxygène par rapport à la somme des organiques introduits. La conversion du HCFO-1233xf obtenue au cours du temps est donnée dans le tableau 1 ci-dessous. Au cours de cet essai, le flux gazeux sortant de la tête de la colonne à distiller est analysé par chromatographie phase gazeuse. L'analyse est reportée dans le tableau 2 ci-dessous (valeur en % d'aire GC).

### Exemple 2 - résultats catalytiques sans air

On reprend le mode réalisation de l'exemple 1 mais sans ajout d'oxygène complémentaire dans la phase gazeuse. Les résultats obtenus pour la conversion au cours du temps sont donnés dans le tableau 1 ci-dessous. L'analyse du flux gazeux sortant de la colonne à distiller est reportée dans le tableau 2 ci-dessous (valeur en % d'aire GC). Les oxydes de carbone et les impuretés en C1 et C2 ont nettement diminué. La pureté de la somme du produit voulu HFO-1234yf et du sous-produit recyclable HFC-245cb augmente.

**Tableau 1**

| Exemple 1 | | Exemple 2 | |
|---|---|---|---|
| Temps (h) | Conversion de HCFO-1233xf (%) | Temps (h) | Conversion de HCFO-1233xf (%) |
| 4 | 78,6 | 15 | 77,6 |
| 8 | 77,4 | 19 | 78,5 |
| 12 | 76,3 | 24 | 77,8 |
| 16 | 77,2 | 27 | 78,3 |
| 21 | 78,7 | 31 | 76,9 |
| 28 | 76,2 | 35 | 74,5 |
| 32 | 76,8 | 39 | 72,8 |
| 36 | 76,9 | 43 | 71,7 |
| 40 | 75,9 | 48 | 72,7 |
| 48 | 75,6 | 51 | 72,9 |
| 52 | 73,4 | 55 | 73,2 |
| 60 | 73,4 | 59 | 73,6 |
| 64 | 72,1 | 63 | 74,1 |
| 71 | 70,2 | 71 | 70,9 |
| 80 | 67,8 | 82 | 70,9 |
| 84 | 65,0 | 86 | 69,4 |

**Tableau 2**

| Produit détecté | Exemple 1 | Exemple 2 |
|---|---|---|
| CO | 3,2 | 0,22 |
| CO₂ | 1,39 | 0,04 |
| F23 | 0,13 | Nd |
| F41 | 0,06 | Nd |
| F32 | 0,03 | Nd |
| F125 | 0,17 | Nd |
| Trifluoropropyne | 0,08 | 0,02 |
| F143a | 0,36 | 0,04 |
| F1234yf + 245cb | 93,19 | 98,03 |
| F40 | 0,26 | Nd |
| F152a | 0,02 | Nd |
| F1234zeE | 1,10 | 1,62 |
| F 1233xf | 0,01 | Nd |

| | | |
|---|---|---|
| *Nd : non détecté* | | |

## Revendications

1. Composition comprenant du tétrafluoropropène **caractérisée en ce qu'**elle comprend, en proportions molaires, de 1 à 50 ppm de HCC-40, de 1 à 50 ppm de HFC-152a, de 1 à 50 ppm de HFC-41 et de 1 à 50 ppm de HFC-32.

## Patentansprüche

1. Zusammensetzung, umfassend Tetrafluorpropen, **dadurch gekennzeichnet, dass** sie in molaren Anteilen 1 bis 50 ppm H-CKW 40, 1 bis 50 ppm H-FKW 152a, 1 bis 50 ppm H-FKW 41 und 1 bis 50 ppm H-FKW 32 umfasst.

## Claims

1. Composition comprising tetrafluoropropene, **characterized in that** it comprises, in molar proportions, from 1 to 50 ppm of HCC-40, from 1 to 50 ppm of HFC-152a, from 1 to 50 ppm of HFC-41 and from 1 to 50 ppm of HFC-32.
